# EUROPEAN PATENT APPLICATION

(11) **EP 1 654 987 A1**
(43) Date of publication of application: **10.05.2006**
(21) Application number: 04771648.5
(22) Date of filing: 06.08.2004
(51) Int. Cl.: A61B 8/00

(54) **ULTRASONOGRAPHIC DIAGNOSTIC EQUIPMENT**

(30) Priority: 14.08.2003 JP 2003293547
(71) Applicant: MATSUSHITA ELECTRIC INDUSTRIAL CO., LTD., Kadoma-shi, Osaka 571-8501 (JP)
(72) Inventor: NISHIGAKI, Morio, Kanagawa 251-0028 (JP)
(74) Representative: Gassner, Wolfgang
(86) International application number: PCT/JP2004/011681
(87) International publication number: WO 2005/016150

(57) **Abstract**

According to the present invention, provided is an ultrasonic diagnostic equipment which meanders the reception focus position so that the reception focus position is made further away from the transmitted beam at the transmission focus depth d1 and is made closer to the transmitted beam in areas shallower and deeper than the depth d1, and thereby makes the shapes of the first and the second composite beams straight lines, thereby preventing the stripe patterns in the display image from being generated and enabling a preferable image quality with reduced image distortion to be obtained, even when the parallel reception is performed.

## Description

### TECHNICAL FIELD

The present invention relates to an ultrasonic diagnostic equipment which performs transmission and reception using an array element to obtain information on a body to be examined.

### BACKGROUND ART

A principle of an ultrasonic diagnostic equipment which repeatedly performs transmissions into and receptions from, of ultrasonic waves, a body to be examined using an array element, thereby to obtain the body information to be examined as a two-dimensional image, is already known.

A tomographic image which is formed by scanning the two-dimensional surface using the ultrasonic beam is called a frame, and an index indicating how many cross-sections of the frames can be displayed for one second is called a frame rate. For example, a frame rate of 15 frame/s means that 15 cross-sections of tomographic images can be obtained for one second. It is known that an image is seen flickering at a frame rate smaller than 30 frame/s, based on characteristics of the human eye. A display frame rate depends on a sonic speed of the ultrasonic wave in a living organism, the number of ultrasonic lines constituting a display, a scan depth and the like.

As a method for improving the display frame rate, a parallel reception method is known (for example, refer to the Japanese Examined Patent Publication No. 56-020017). Hereinafter, the prior art will be described with reference to FIG. 6, FIG. 7A and FIG. 7B.

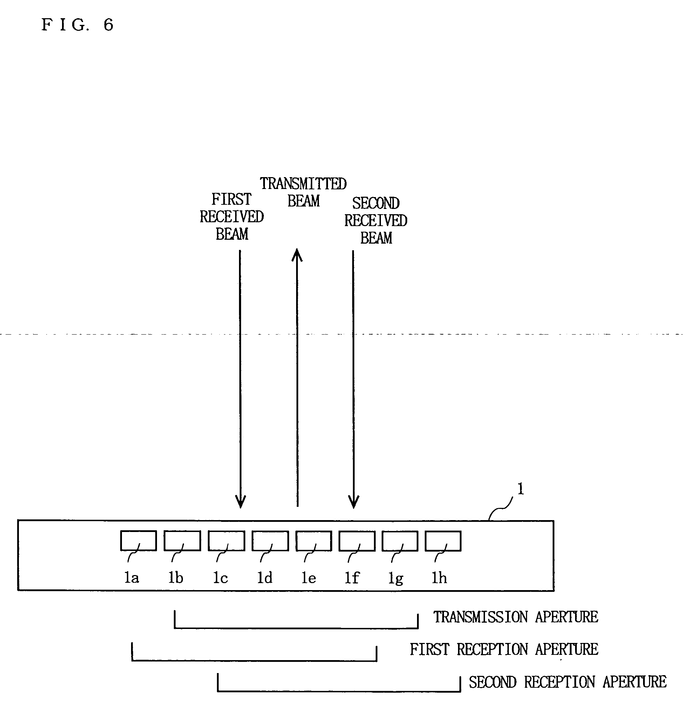

FIG. 6 is a diagram for explaining a prior art parallel reception method and illustrating a configuration of an array element. In FIG. 6, a plurality of elements are aligned in an array element 1, and the elements 1a to 1h used for the transmission and reception among them are shown.

The elements 1b to 1g are used for forming a transmitted beam and emit ultrasonic waves into a body to be examined not shown. The transmitted beam propagates from the intermediate position between the elements 1d and 1e along a straight line perpendicular to the array direction of the elements. In this example, for reception, two received beams (a first and a second received beams) are formed so as to receive information from the respective points on the two parallel straight lines sandwiching the transmitted beam therebetween. The first received beam is formed using the elements 1a to 1f as a first reception aperture and the second received beam is formed using the elements 1c to 1h as a second reception aperture. The first received beam propagates to the intermediate position between the elements 1c and 1d and the second received beam propagates to the intermediate position between the elements 1e and 1f along the straight lines perpendicular to the array direction of the elements, respectively.

As a result, a directivity of the transmitted beam - the first received beam for the transmission and reception is positioned from the position of the element 1d in the direction perpendicular to the array direction of the elements while a directivity of the transmitted beam - the second received beam for the transmission and reception is positioned from the position of the element 1e in the direction perpendicular to the array direction of the elements.

When the scanning is sequentially performed using two received beams with one transmitted beam as described above, as compared to a case where the scanning is sequentially performed using one received beam with one transmitted beam, a capture time of image data for each screen can be shortened, thereby enabling the frame rate to be improved.

While in the above-described prior art the transmission aperture position is displaced with respect to the reception aperture position, thereby displacing the transmitted beam position with respect to the received beam position, the parallel reception can be performed also by using the same aperture for the transmission and reception to deflect the received beams.

While the above description is given of the linear scanning, the parallel reception is possible with using the same principle for the electronic sector scanning. In the case of the sector scanning, the same aperture is used for the transmission and reception to change the beam deflection angles of both the transmitted beam and the plurality of received beams, thereby performing the parallel reception (for example, refer to the Japanese Laid-open Patent Publication No. 2000-254120).

### DISCLOSURE OF THE INVENTION

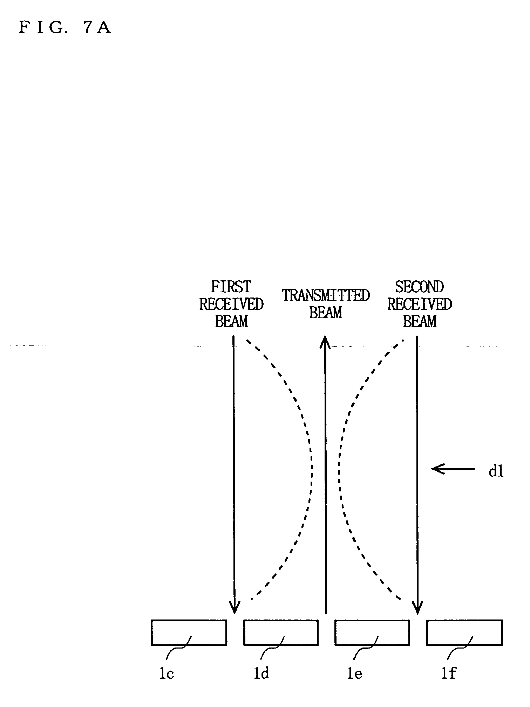

However, in practice, in the prior art shown in FIG. 6, while the first and the second received beams are constantly narrowed through dynamic focusing, the transmitted beam is narrowed in the vicinity of the focus d1 and widened in the shallower area and the deeper area than the focus as shown by the dotted lines in FIG. 7A.

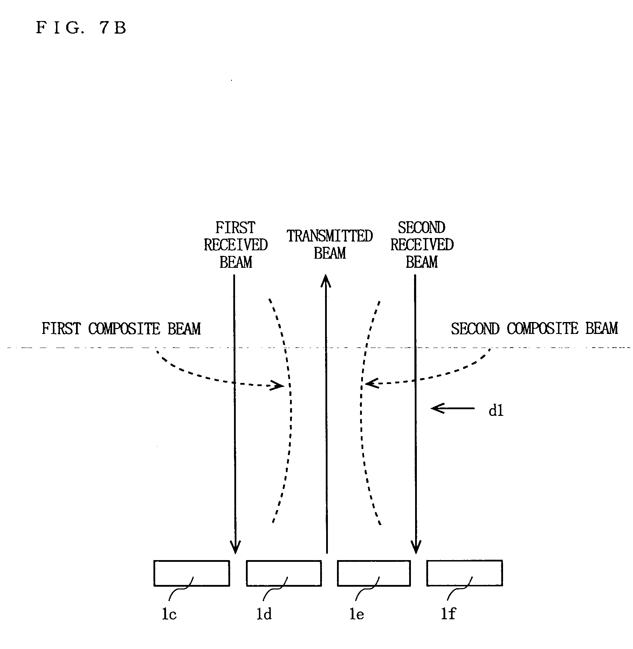

Therefore, a first composite beam of the transmitted beam and the first received beam and a second composite beam of the transmitted beam and the second received beam deflect toward the transmitted beam side in the vicinity of the focus of the transmitted beam and deflect toward the first and the second received beam sides, respectively, in the shallower area and the deeper area than the focus as shown by the dotted lines in FIG. 7B and thereby there is a problem that the first and the second composite beams are not made parallel to each other and when the image is displayed, stripe patterns occur in the display image.

The present invention is made in view of the above-described problems and its object is to provide an ultrasonic diagnostic equipment which can prevent the strip patterns in the display image from being generated and obtain a preferable image quality with reduced image distortion when performing parallel reception.

To achieve the above objects, according to the present invention, provided is an ultrasonic diagnostic equipment performing parallel reception controls the directivities of the received beams so as not to make the shapes of the received beams parallel to the transmitted beam, thereby making the composite beams straight lines.

A first aspect of the present invention is directed to provide an ultrasonic diagnostic equipment in which when the parallel reception is performed with linear scanning using an array element, a movement track of focus points in reception dynamic focusing is meandered in relation to transmission focus positions so that a composite beam of a received beam and a transmitted beam is substantially shaped as a straight line.

According to this feature, in the linear scanning, the positions of the reception focuses are meandered so as to be moved further away from the transmitted beam at the transmission focus depth and be moved closer to the transmitted beam in the areas shallower and deeper than the transmission focus depth, thereby enabling the composite beams to be shaped as straight lines.

A second aspect of the present invention is directed to provide an ultrasonic diagnostic equipment in which when the parallel reception is performed with linear scanning using an array element, a movement track of focus points in reception dynamic focusing is moved in the slanting straight line direction with respect to the transmission direction in relation to transmission focus positions so that a composite beam of a received beam and a transmitted beam is substantially shaped as a straight line at least in areas having shallower depths than the focus position of the transmitted beam.

According to this feature, in the linear scanning, the positions of the reception focuses are moved closer to the transmitted beam in the area of shallower depth and moved further away from the transmitted beam in the area of deeper depth, thereby enabling the shapes of the composite beams to be close to straight lines.

A third aspect of the present invention is directed to provide an ultrasonic diagnostic equipment in which when the parallel reception is performed with sector scanning using an array element, a movement track of focus points in reception dynamic focusing is meandered in relation to transmission focus positions so that a composite beam of a received beam and a transmitted beam is substantially shaped as a straight line.

According to this feature, in the sector scanning, as compared to the original positions, the positions of the reception focuses are meandered so as to be moved further away from the transmitted beam at the transmission focus depth and be moved closer to the transmitted beam in the areas shallower and deeper than the transmission focus depth, thereby enabling the composite beams to be shaped as straight lines.

A fourth aspect of the present invention is directed to provide an ultrasonic diagnostic equipment in which when the parallel reception is performed with sector scanning using an array element, a movement track of focus points in reception dynamic focusing is moved in the slanting straight line direction with respect to the transmission direction in relation to transmission focus positions so that a composite beam of a received beam and a transmitted beam is substantially shaped as a straight line at least in areas having shallower depths than the focus position of the transmitted beam.

According to this feature, in the sector scanning, as compared to the original positions, the positions of the reception focuses are moved closer to the transmitted beam in the area of shallower depth and moved further away from the transmitted beam in the area of deeper depth, thereby enabling the shapes of the composite beams to be close to straight lines.

Preferably, in the respective ultrasonic diagnostic equipments according to the present invention, the movement of the focus points is performed by controlling delay times corresponding to the respective elements constituting the array element.

According to this feature, the control of the received beam propagation is realized by controlling the delay time obtained when the delays in the respective receptions are added, thereby enabling the shapes of the composite beams to be easily made close to straight lines.

Preferably, in the respective ultrasonic diagnostic equipments according to the present invention, the movement of the focus points is performed by controlling gains of the receiving circuit, the gains corresponding to the respective elements constituting the array element, or controlling both the gains of the receiving circuit and the delay times.

According to this feature, the control of the received beam propagation is realized by controlling the weighting for the reception gains, thereby enabling the shapes of the composite beams to be easily made close to straight lines.

Preferably, in the respective ultrasonic diagnostic equipments according to the present invention, the array element is a two-dimensional array element.

According to this feature, also when three-dimensional scanning is performed using the two-dimensional array element, the control of the received beam propagation is performed, thereby enabling the shapes of the composite beams to be close to straight lines.

According to the present invention, an ultrasonic diagnostic equipment which can prevent the stripe patterns in the display image from being generated and obtain a preferable image quality with reduced image distortion even when performing parallel reception, can be provided.

### BRIEF DESCRIPTION OF THE DRAWINGS

FIG. 1 is a diagram illustrating shapes of transmitted and received beams and a positional relationship among them in the linear scanning with an ultrasonic diagnostic equipment according to a first embodiment of the present invention.
FIG. 2 is a diagram illustrating shapes of transmitted and received beams and a positional relationship among them in the linear scanning with an ultrasonic diagnostic equipment according to a second embodiment of the present invention.
FIG. 3 is a diagram illustrating shapes of a transmitted beam and a received beam weighted by gain control and a positional relationship therebetween in the linear scanning with an ultrasonic diagnostic equipment according to a third embodiment of the present invention.
FIG. 4A is a diagram illustrating shapes of transmitted and received beams and a positional relationship among them in the sector scanning with an ultrasonic diagnostic equipment according to a fourth embodiment of the present invention.
FIG. 4B is a diagram illustrating shapes of transmitted and received beams and a positional relationship among them in sector scanning according to a prior art as a comparative example for FIG. 4A.
FIG. 5A is a diagram illustrating shapes of transmitted and received beams and a positional relationship among them in a case where two-dimensional sector scanning is performed using a two-dimensional array element with an ultrasonic diagnostic equipment according to a fifth embodiment of the present invention.
FIG. 5B is a diagram illustrating shapes of transmitted and received beams and a positional relationship among them in a case where two-dimensional sector scanning is performed using a two-dimensional array element according to a prior art as a comparative example for FIG.5A.
FIG. 6 is a diagram for explaining parallel reception in a prior art.
FIG. 7A is a diagram for explaining a problem on the parallel reception in the prior art.
FIG. 7B is a diagram for explaining a problem on the parallel reception in the prior art.
FIG. 8 is a block diagram illustrating an outline configuration of an ultrasonic diagnostic equipment 100 adopting an electronic sector scanning method according to an embodiment of the present invention.
FIG. 9 is a block diagram illustrating an example of a configuration of a beam forming unit 10.
FIG. 10 is a block diagram illustrating an outline configuration of an ultrasonic diagnostic equipment 200 adopting an electronic linear scanning method according to an embodiment of the present invention.

### BEST MODE FOR CARRYING OUT THE INVENTION

While hereinafter embodiments of the present invention will be described with reference to the drawings, the present invention is not limited to these embodiments.

### (Ultrasonic diagnostic equipment using electronic sector scanning method)

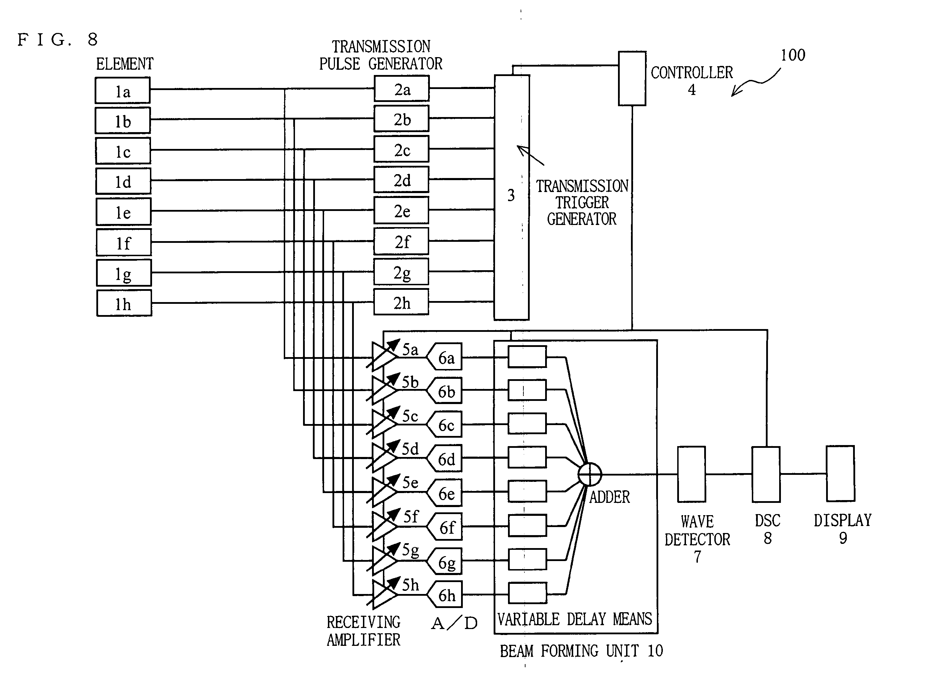

FIG. 8 is a block diagram illustrating an outline configuration of an ultrasonic diagnostic equipment 100 adopting an electronic sector scanning method according to an embodiment of the present invention. As shown in FIG. 8, the ultrasonic diagnostic equipment 100 comprises an array element (elements 1a to 1h), a transmitting circuit (comprising transmission pulse generators 2a to 2h and a transmission trigger generator 3), a receiving circuit (comprising receiving amplifiers 5a to 5h, A/D converters 6a to 6h, and a beam forming unit 10 (including delay means and an adder)), a controller 4, a wave detector 7, a digital scan converter (DSC) 8, and an display 9.

The ultrasonic diagnostic equipment 100 using the electronic sector scanning method uses the same aperture (a portion which actually transmits and receives an ultrasonic wave among an array of the elements) for the transmission and reception and changes directivities of the transmitted and received beams (deflection), thereby performing two-dimensional scanning. The temporal timing is made to change among transmissions from and/or receptions into the elements 1a to 1h (control of the delay times), and thereby the directivities of the transmitted and received beams can be operated.

The transmission trigger generator 3 generates a trigger signal for determining timing for beam transmission under the control of the controller 4. The transmission pulse generators 2a to 2h generate transmission pulses for driving the elements 1a to 1h on the basis of the trigger signals, respectively. The transmission pulses are generated at independent timings by the transmission pulse generators 2a to 2h, respectively, to obtain a desired directivity.

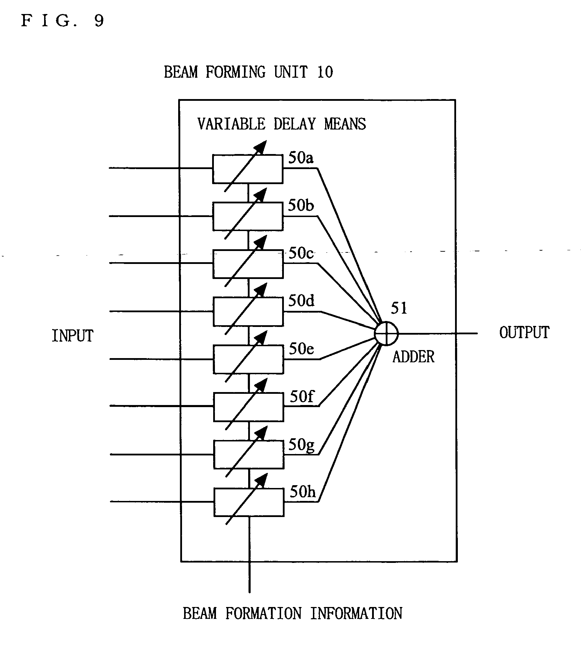

Further, the same can be said for received echo. FIG. 9 illustrates an example of an internal configuration of the beam forming unit 10 in more detail. The beam forming unit 10 for the received beam includes variable delay means 50a to 50h for delaying the echo signals obtained by the elements 1a to 1h as desired, respectively, and an adder 51 for adding the signals from the variable delay means 50a to 50h to obtain data of a desired directivity. Under the control of the controller 4, the variable delay means 50a to 50h delay the echo signals of the respective signals and the adder 51 adds the delayed signals together, thereby enabling the echo signal from the desired direction to be obtained.

At the reception, as described above, the phases of the ultrasonic waves can be controlled so as to be constantly changed while the ultrasonic echoes are being received. That is, the beam can be dynamically changed so that while phase control is performed so as to focus the beam at a short distance near the start of the reception, the beam is focused in the distance with the passage of reception time. Thereby, the received beam can be made narrow along any distance from the short distance to the long distance. This is called dynamic focusing.

As described above, the delay times are controlled also in the case of reception, thereby enabling the echo signals to have the directivities. Further, the receiving amplifiers 5a to 5h change gains, thereby enabling the directivities of the received beams to be changed.

Next, the ultrasonic signal which is obtained from the addition by the adder 51 is subjected to envelope detection by the wave detector 7 and then transmitted to the DSC 8, and transformed by the DSC 8 into a scanning line for the display on the basis of the control of the controller 4, and thereafter displayed as a two-dimensional image by the display 9.

As described above, the ultrasonic diagnostic equipment 100 using the electronic sector scanning method electrically controls the directivities of the transmitted and received beams, thereby obtaining a two-dimensional image of a scanned portion of a body to be examined.

### (Ultrasonic diagnostic equipment using electronic linear scanning method)

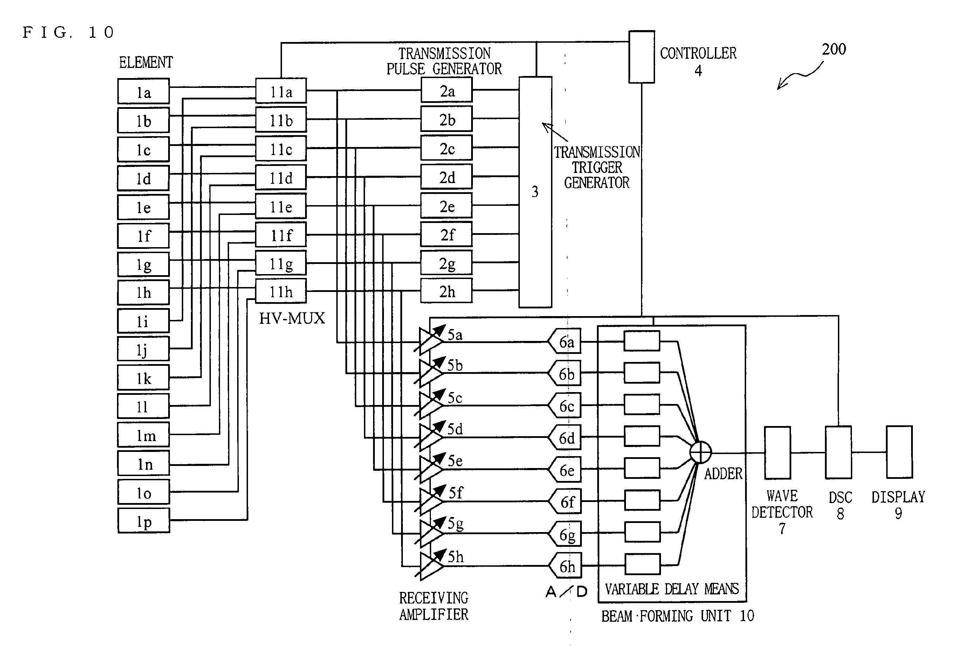

On the other hand, FIG. 10 is a block diagram illustrating an outline configuration of an ultrasonic diagnostic equipment 200 adopting an electronic linear scanning method according to an embodiment of the present invention. As shown in FIG. 10, the ultrasonic diagnostic equipment 200 using the electronic linear scanning method is different in configuration than the ultrasonic diagnostic equipment 100 using the electronic sector scanning method in that the ultrasonic diagnostic equipment 200 is provided with high breakdown voltage switches (HV-MUX: High Voltage MultiPlexer) 11a to 11h for selecting elements, between the elements 1a to 1p and the transmission pulse generators 2a to 2h (or the receiving amplifiers 5a to 5h).

The HV-MUXs 11a to 11h each has two channels (it is assumed that they are channel 1 and channel 2) for selecting one of two elements which are assigned to each HV-MUX. Forexample, the HV-MUX 11a has the channel 1 connected to the element 1a and the channel 2 connected to the element 1i, and can select one of the elements on the basis of the control of the controller 4. The same can be said for the other HV-MUXs 11b to 11h.

In the electronic linear scanning method, unlike in the case of the electronic sector scanning method, no deflection is performed and the transmission aperture positions are displaced with respect to the reception aperture positions little by little, thereby performing two-dimensional scanning.

For example, in the first transmission and reception, the HV-MUXs 11a to 11h each selects the channel 1 to perform the transmission and reception using the elements 1a to 1h. In the subsequent transmission and reception, the HV-MUX 11a switches to the channel 2 (channel 1 remains being selected by all the other HV-MUXs). Thereby, the selected elements are 1b to li and the aperture is shifted by one element.

As described above, the HV-MUXs sequentially perform the switching to scan a portion to be examined with changing the apertures, thereby obtaining a two-dimensional image of the portion to be examined.

On the basis of the control of the controller 4, the delay means in the beam forming unit 10 perform delaying and the adder 51 performs addition, thereby enabling the directivities of the received beams which are received by the elements 1a to 1p to be changed, as in the case of the electronic sector scanning method. Further, the directivities of the received beams can be changed also by changing gains by means of the receiving amplifiers 5a to 5h.

Next, the ultrasonic signal which is obtained from the addition by the adder 51 is subjected to envelope detection by the wave detector 7 and then transmitted to the DSC 8, and transformed by the DSC 8 into a scanning line for the display under the control of the controller 4, and thereafter displayed as a two-dimensional image by the display 9.

While above-described are the typical configurations of the ultrasonic diagnostic equipment using the electronic linear scanning method and the ultrasonic diagnostic equipment using the electronic sector scanning method, these are only illustrative, and it is apparent that various variation can be similarly used for the object of the present invention.

Hereinafter, the preferred embodiments of the present invention in the case of obtaining a two-dimensional image of a body to be examined using the ultrasonic diagnostic equipment using either the electronic linear scanning method or the electronic sector scanning method will be described with reference to the drawings.

### (Embodiment 1)

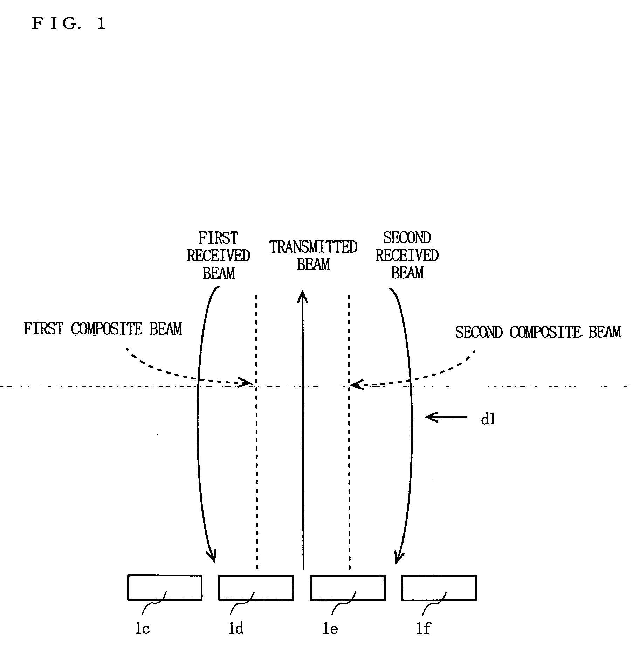

FIG. 1 is a diagram illustrating shapes of transmitted and received beams and a positional relationship among them in the ultrasonic diagnostic equipment performing parallel reception through linear scanning using the array element according to a first embodiment of the present invention.

In FIG. 1, in a case where a transmitted beam propagates from the intermediate position between the elements 1d and 1e, a first received beam propagates to the intermediate position between the elements 1c and 1d, and a second received beam propagates to the intermediate position between the elements 1e and 1f , it is desired that a first composite beam is disposed at the position of the element 1d and a second composite beam is disposed at the position of the element 1e. At this time, in order to prevent the first and the second composite beams from deflecting toward the transmitted beam side in the vicinity of the transmission focus depth, the directivities of the first and the second received beams are controlled by changing the delay times so that the beam positions of the first and the second received beams are moved further away from that of the transmitted beam in the vicinity of the transmission focus depth d1 as shown in FIG. 1. Thereby, the first and the second composite beams are made straight lines as shown by the dotted lines.

As described above, according to this embodiment, even when the parallel reception is performed, the composite beams of the transmission and reception can be aligned parallel to each other, and as a result the stripe patterns in the display image can be prevented from being generated and a preferable image with reduced image distortion can be obtained. Further, since the distortion in the image is reduced, for example, when a length is measured using a caliper function, the problem that the measured data become different due to a subtle deviation in the positional relationship between a body to be examined and a probe is avoided.

### (Embodiment 2)

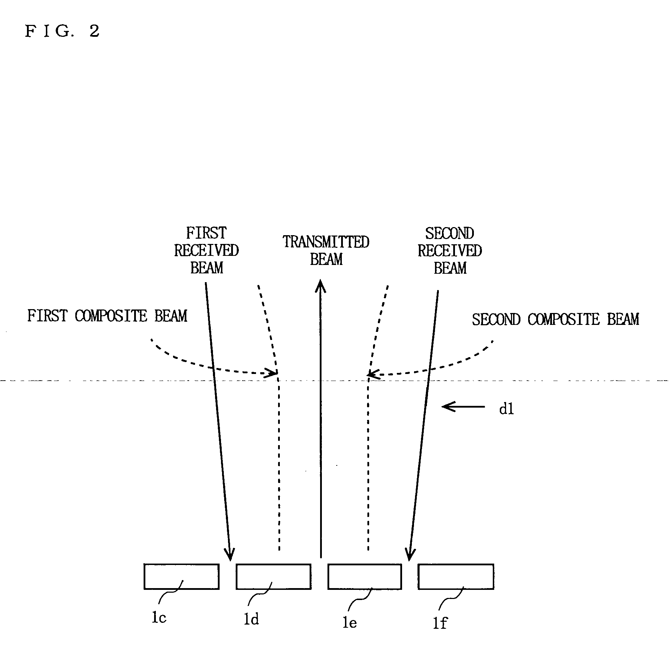

FIG. 2 is a diagram illustrating shapes of the transmitted and received beams and a positional relationship among them in the ultrasonic diagnostic equipment performing parallel reception through linear scanning using the array element according to a second embodiment of the present invention.

In FIG. 2, in a case where the transmitted beam propagates from the intermediate position between the elements 1d and 1e, the first received beam propagates to the intermediate position between the elements 1c and 1d, and the second received beam propagates to the intermediate position between the elements 1e and 1f, it is desired that the first composite beam is disposed at the position of the element 1d and the second composite beam is disposed at the position of the element 1e. At this time, in order to prevent the first and the second composite beams from deflecting toward the transmitted beam side in the vicinity of the transmission focus depth, the directivities of the first and the second received beams are controlled by changing the delay times by the receiving circuit so that the beam positions of the first and the second received beams are moved further away from that of the transmitted beam in the vicinity of the transmission focus depth d1 as shown in FIG. 2.

At this time, unlike in the case shown in FIG. 1, the received beam is controlled by setting the delay times so that the received beam is in a shape of a straight line, such that from a position of shallower depth toward a position of deeper depth, the distances between the received beams and the transmitted beam are greater as shown in FIG. 2, and consequently the first and the second composite beams are close to straight lines perpendicular to the array direction of the elements from the area of shallower depth toward the focus depth of the transmitted beam. The attenuation of signals is increased in the area of deeper depth than the transmission focus, and the images are not required to have sharpness in many cases, and therefore it is not always necessary that the first and the second composite beams are parallel to each other. As described above, when the received beams are controlled so as to become straight lines, it is advantageous that the calculation of focus for reception is facilitated.

As described above, according to this embodiment, even when the parallel reception is performed, the composite beams of the transmission and reception can be aligned parallel to each other at least from the focus depth of the transmitted beam toward the position of shallower depth, and thereby the stripe patterns in the display image can be prevented from being generated and a preferable image with reduced image distortion can be obtained. Further, since the distortion in the image is reduced, for example, when a length is measured using a caliper function, the problem that the measured data become different due to a subtle deviation in the positional relationship between a body to be examined and a probe is avoided.

### (Embodiment 3)

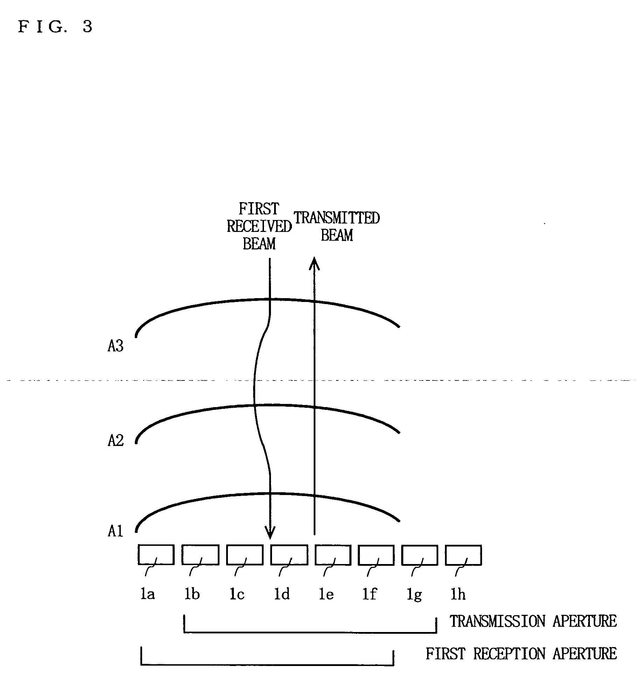

FIG. 3 is a diagram for explaining and schematically showing that the reception focus positions are controlled by controlling gains of the receiving circuit in the ultrasonic diagnostic equipment performing parallel reception through linear scanning using the array element according to a third embodiment of the present invention. In FIG. 3, only the transmitted beam and the first received beam are illustrated and the second received beam is omitted.

In FIG. 3, thick lines A1, A2, and A3 drawn above the elements 1a to 1f indicate gains of the receiving circuit, which correspond to the respective elements, that is, weighting. For example, as to the thick line A1, the thick line part corresponding to the position of the element 1c is positioned higher in the figure than the thick line part corresponding to the position of the element 1a, which indicates that the element 1c has a higher gain of the receiving circuit than the element 1a. Further, A1, A2, and A3 are indicated in order starting from the position of shallower beam depth toward the position of deeper beam depth, which indicates that A1 to A3 correspond to the gains of the received beam of the receiving circuit at the respective depths.

As indicated as A1 which is the area of shallower beam depth and A3 which is the area of deeper beam depth, the weighting is uniformly performed on the left and the right, and therefore the received beam propagates to the center of the first reception aperture on the straight line perpendicular to the array direction of the elements. On the other hand, the weighting A2 is unbalancedly heavier on the left at the focus depth of the transmitted beam, and the propagation of the received beam is deflected toward the left and is moved further away from the transmitted beam.

Accordingly, as a whole, the first received beam has the directivity such that the first received beam is moved further away from the transmitted beam and has a meandering shape in the vicinity of the focus depth of the transmitted beam as shown in FIG. 3, and the composite beam of the first received beam and the transmitted beam is disposed from the vicinity of the element 1d on the straight line perpendicular to the array direction of the elements. The same can be said for the composite beam of the second received beam (not shown) and the transmitted beam. Then, the adjustment of the weighting of the received beams can be performed by the receiving amplifiers 5a to 5h (refer to FIG. 10) under the control of the controller 4.

As described above, according to this embodiment, the shapes of the composite beams of the transmission and reception are maintained as approximate straight lines and can be aligned parallel to each other, and thereby the stripe patterns in the display image can be prevented frombeing generated and a preferable image with reduced image distortion can be obtained. Further, since the distortion in the image is reduced, for example, when a length is measured using a caliper function, the problem that the measured data become different due to a subtle deviation in the positional relationship between a body to be examined and a probe is avoided.

Then, while in this embodiment an example of adjusting the directivities of the received beams by adjusting the gains of the receiving circuit is illustrated, both the gains of the receiving circuit and the delay times of the receiving circuit may be controlled so as to adjust the directivities of the received beams (the same can be said for the other embodiments.)

### (Embodiment 4)

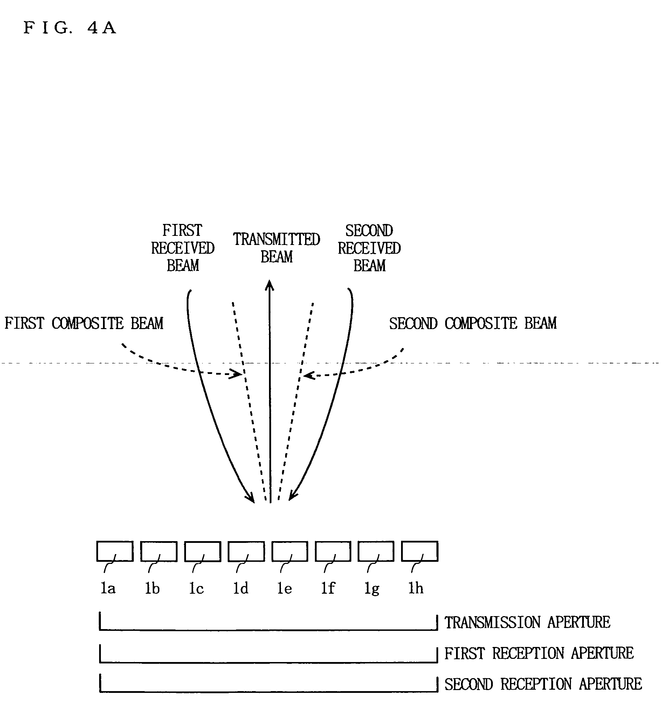

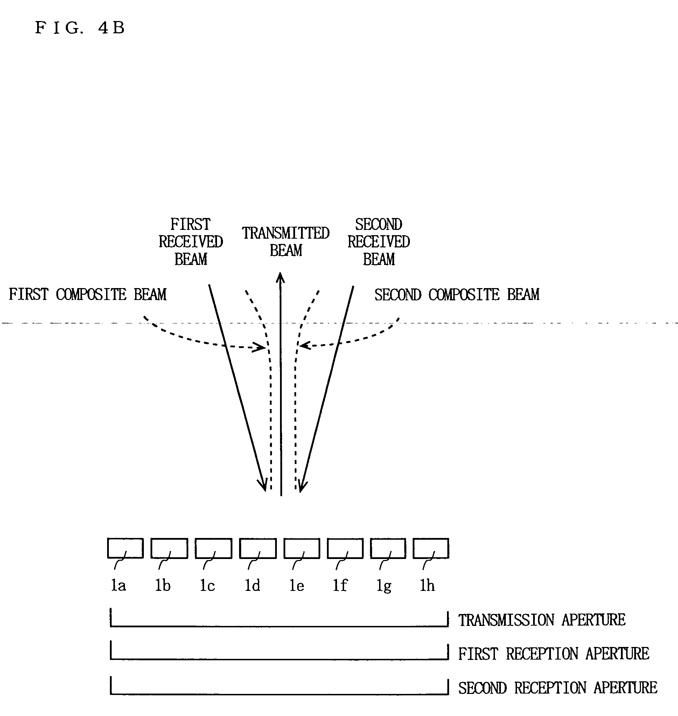

FIG. 4A is a diagram for explaining and schematically illustrating controls of the received beams in the ultrasonic diagnostic equipment performing parallel reception through sector scanning using the array element according to a fourth embodiment of the present invention, while FIG. 4B is a diagram schematically illustrating controls of the received beams in a prior art ultrasonic diagnostic equipment performing parallel reception through sector scanning using an array element as a comparative example.

In the sector scanning, the aperture center position is the same between the transmission aperture and the reception aperture. In the prior art shown in FIG. 4B, the first and the second received beams are straight lines, and therefore the first and the second composite beams (dotted lines) of the transmission and reception are curved so as to be made closer to the transmitted beam at the transmission focus depth.

On the other hand, according to this embodiment shown in FIG. 4A, in order to make the first and the second composite beams of the transmission and reception straight lines as shown by the dotted lines in the FIG., the delay times obtained when the delays of the received beams are added are controlled so that the first and the second received beams are moved further away from the transmitted beam at the focus depth of the transmitted beam as compared to the prior art.

As described above, according to this embodiment, the composite beams can be equally spaced, and thereby the stripe patterns in the display image can be prevented from being generated and a preferable image with reduced image distortion can be obtained. Further, since the distortion in the image is reduced, for example, when a length is measured using a caliper function, the problem that the measured data become different due to a subtle deviation in the positional relationship between a body to be examined and a probe is avoided.

### (Embodiment 5)

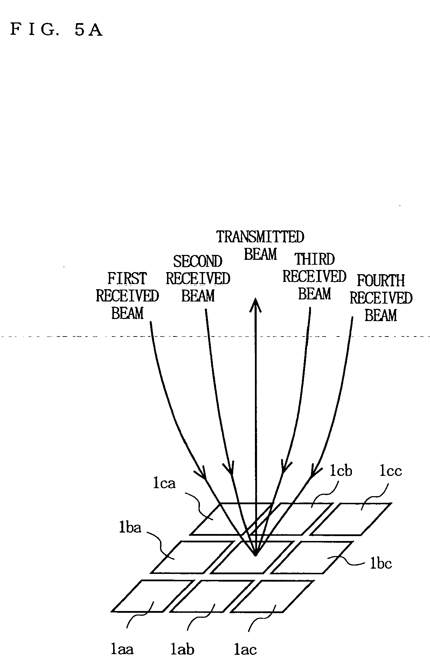

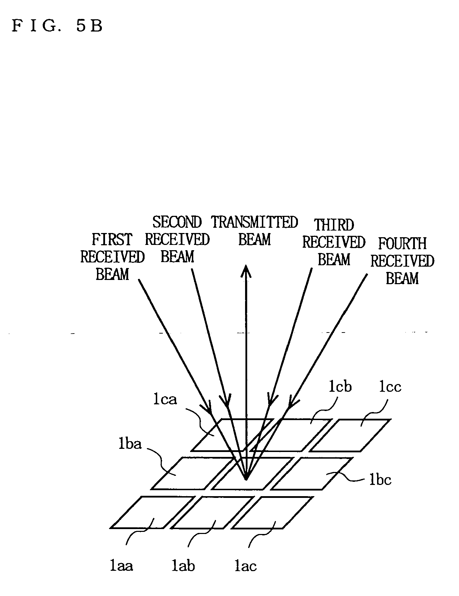

FIG. 5A is a diagram for explaining and schematically illustrating controls of the received beams through two-dimensional sector scanning using a two-dimensional array element in the ultrasonic diagnostic equipment according to a fifth embodiment of the present invention, while FIG. 5B is a diagram schematically illustrating controls of the received beams in a prior art ultrasonic diagnostic equipment as a comparative example.

In the sector scanning, the aperture center position is the same between the transmission aperture and the reception aperture. In the prior art shown in FIG. 5B, the first to the fourth received beams are straight lines, and therefore the composite beams of the transmission and reception are curved so as to be made closer to the transmitted beam at the transmission focus depth.

On the other hand, according to this embodiment shown in FIG. 5A, in order to make the composite beams of the transmission and reception straight lines, the delay times obtained when the delays of the received beams are added are controlled so that the first to the fourth received beams are moved further away from the transmitted beam at the focus depth of the transmitted beam as compared to the prior art.

As described above, according to this embodiment, the composite beams can be equally spaced, and thereby the stripe patterns in the display image can be prevented from being generated and a preferable image with reduced image distortion can be obtained. Further, since the distortion in the image is reduced, for example, when a length is measured using a caliper function, the problem that the measured data become different due to a subtle deviation in the positional relationship between a body to be examined and a probe is avoided.

Then, while in this embodiment a case where the sector scanning is performed in both of the two dimensions is illustrated and described, the similar method is applicable when the sector scanning is performed in one dimension and the linear scanning is performed in the other dimension.

### INDUSTRIAL APPLICABILITY

The ultrasonic diagnostic equipment according to the present invention is advantageous in that the composite beams are controlled so as to be made straight lines even when the parallel reception is performed, thereby preventing the stripe patterns in the display image from being generated and enabling a preferable image quality with reduced image distortion to be obtained, and is applicable to medical applications and the like.

## Claims

1. An ultrasonic diagnostic equipment which performs parallel reception, wherein when the parallel reception is performed with linear scanning using an array element, a movement track of focus points in reception dynamic focusing is meandered in relation to transmission focus positions so that a composite beam of a received beam and a transmitted beam is substantially shaped as a straight line.

2. An ultrasonic diagnostic equipment which performs parallel reception, wherein when the parallel reception is performed with linear scanning using an array element, a movement track of focus points in reception dynamic focusing is moved in the slanting straight line direction with respect to the transmission direction in relation to transmission focus positions so that a composite beam of a received beam and a transmitted beam is substantially shaped as a straight line at least in areas having shallower depths than the focus position of the transmitted beam.

3. An ultrasonic diagnostic equipment which performs parallel reception, wherein when the parallel reception is performed with sector scanning using an array element, a movement track of focus points in reception dynamic focusing is meandered in relation to transmission focus positions so that a composite beam of a received beam and a transmitted beam is substantially shaped as a straight line.

4. An ultrasonic diagnostic equipment which performs parallel reception, wherein when the parallel reception is performed with sector scanning using an array element, a movement track of focus points in reception dynamic focusing is moved in the slanting straight line direction with respect to the transmission direction in relation to transmission focus positions so that a composite beam of a received beam and a transmitted beam is substantially shaped as a straight line at least in areas having shallower depths than the focus position of the transmitted beam.

5. The ultrasonic diagnostic equipment according to any of Claims 1 to 4, wherein the movement of the focus points is performed by controlling delay times corresponding to the respective elements constituting the array element.

6. The ultrasonic diagnostic equipment according to any of Claims 1 to 4, wherein the movement of the focus points is performed by controlling gains of the receiving circuit, the gains corresponding to the respective elements constituting the array element, or controlling both the gains of the receiving circuit and the delay times.

7. The ultrasonic diagnostic equipment according to any of Claims 3 to 6, wherein the array element is a two-dimensional array element.
